# EUROPEAN PATENT APPLICATION

(11) **EP 4 130 293 A1**
(43) Date of publication of application: **08.02.2023**
(21) Application number: 21189685.7
(22) Date of filing: 04.08.2021
(51) Int. Cl.: C12Q 1/6806, C12Q 1/6886, G16B 30/00

(54) **METHOD OF MUTATION DETECTION IN A LIQUID BIOPSY**

(71) Applicant: OncoDNA SA, 6041 Gosselies (BE)
(72) Inventor: LAES, Jean-François, 1400 Nivelles (BE)
(74) Representative: Calysta NV

(57) **Abstract**

Method to detect a mutation from a liquid biopsy of a patient, the method comprises the steps of: collecting DNA fragments from said liquid biopsy, preparing a DNA library of the DNA fragments, sequencing the DNA library, identifying DNA variant fragments of the DNA fragments having a mutation, associating the identified DNA variant fragments to a first group and to a second group based on the length of the DNA variant fragments, detecting, if the mutation is tumorous or healthy based on the presence of the DNA variant fragments in the first group and in the second group.

## Description

### Technical domain

The present disclosure concerns analysis linked to genome. In particular, the discovery of circulating cell-free DNA (cfDNA) has provided the opportunity to analyze genetic materials from a liquid biopsy without the risks associated with invasive sampling methods.

More specifically, but not exclusively, the present disclosure refers to a method to detect a mutation from a liquid biopsy of a patient to a method to identify, to follow and/or to adapt a treatment adapted to a mutational profile of a tumor of the patient and to a theranostic report of the tumor of the patient.

### State of the art

Small quantities of circulating tumor DNA (ctDNA) originating from tumors, especially malignant tumors, can be found among the cfDNA in the blood of cancer patients. Assays that detect ctDNA from blood in order to diagnose or better identify a disease are known as liquid biopsies. Currently, some limitations are present in existing methods in liquid biopsies for cancer diagnosis, especially in early cancer stages, ctDNA is present in very low levels as compared to cfDNA in the blood. As a consequence, the sensitivity of most existing methods is not high enough to detect ctDNA in an early stage or the methods are so complex that they cannot be used in the clinical practice.

It is further known that the average length of ctDNA fragments from tumor cells are shorter than the cfDNA fragments from normal, healthy, cells that are unaffected by cancers. Different documents are known to provide methods for increasing the sensitivity of detecting a certain ctDNA from a liquid biopsy.

Document WO2020/94775 provides a computer-implemented method for detecting variant nucleic acid from a cell-free nucleic acid containing sample. The method comprises: a) providing data representing fragment sizes of nucleic acid fragments obtained from said sample and/or representing a measure of deviation from copy number neutrality of the nucleic acid fragments obtained from said sample; b) causing a processor of the computer to process the data from step a) according to a classification algorithm that has been trained on a training set comprising a plurality of samples of cell-free nucleic acid containing the variant nucleic acid and a plurality of samples not containing the variant nucleic acid, wherein said classification algorithm operates to classify sample data into one of at least two classes, the at least two classes comprising a first class containing the variant nucleic acid and a second class not containing the variant nucleic acid, based on a plurality of cell-free nucleic acid fragment size features and/or a deviation from copy number neutrality feature; and c) outputting the classification of the sample from step b) and thereby determining whether the sample contains the variant nucleic acid or not, or determining a probability that the sample contains the variant nucleic acid.

Document US20200294624 provides a sensitive, fast, and less expensive method for classifying a cancer using size-selected cell-free DNA sequence. The method described in this document allows enriching the fraction of cancer-derived cfDNA fragments in cancer samples from all types of cancers, including low tumor-derived cfDNA cancers. Further, the methodologies facilitate classification of cancers having very low tumor fractions (e.g., early-stage cancers and low ctDNA cancers), even below 1% tumor fraction. The method proposes to take a sample from an affected region and a liquid sample of an unaffected region. A histogram of the DNA fragment lengths of the two samples are created. Based on the ratio of the two histograms, the fragment size bins are ranked according to a high concentration of cfDNA fragments of the tumorous sample with respect to the cfDNA of the non-tumorous sample. A particular ctDNA known to be cancerous can then be detected in the liquid sample based on the highest ranked fragment size bins.

Document EP3612964 discloses methods for determining a presence or a copy number of a genetic sequence variant associated with a tumor in a test sample by analyzing sizes and sequences of cfDNA fragments obtained from the test sample. This document provides processes and systems for liquid biopsies that effectively combine size information with sequence information of cfDNA, thereby achieving high analytical sensitivity and specificity for calling tumor-associated variants and determining cancers. According to this document, one aspect of the disclosure relates to methods for determining a presence or a copy number of a genetic sequence variant associated with a tumor in a test sample by analyzing sizes and sequences of cfDNA fragments obtained from the test sample.

These documents exploit the difference in size and sequence information of cfDNA and ctDNA fragments for achieving high analytical sensitivity and specificity for calling tumor-associated variants and determining cancers. This allows to increase the sensitivity for detecting the presence of a specific variant DNA (e.g. one which is known to be cancerous) in a liquid sample, even if the specific variant DNA is present only with low concentration.

Unfortunately, although these documents describe the merits of their technology, currently no method exists allowing to detect if a mutation is tumorous or healthy based on DNA collected from a liquid biopsy of a patient with sufficiently high sensitivity to be applied in routine clinical settings. Indeed, these documents developed methods for determining a presence of a genetic sequence variant associated with a tumor. However, no method exists to identify a previously unknown mutation from a liquid biopsy and which is able to classify this previously unknown mutation as tumorous or healthy. To improve efficacy of cancer treatment and reduce the cost of liquid biopsy analyses, it is indeed not only important to be able to detect mutation from a liquid biopsy with higher sensitivity and to detect the presence of a genetic sequence variant associated with a tumor, but it is also needed to detect if a mutation is tumorous (originating from a tumor cell) or healthy (originating from a normal, healthy, cell) in order to adapt the cancer treatment in the most efficient manner. Indeed, recommended cancer treatment will be different if the same mutation is tumorous or healthy.

### Summary of the invention

There is therefore a need to have a method to achieve an identification of tumorous or healthy mutation based on a liquid biopsy of the patient.

According to the present invention, a method to detect a mutation from a liquid biopsy of the patient according to the independent claims is provided.

While in the state of the art, the DNA variant fragment size was only used to increase the sensitivity for detecting a particular mutation, the inventors found out that the DNA variant fragment size can be used to detect, if a mutation is tumorous or not. Indeed, by analyzing DNA variant fragments in both groups, the method according to the present invention is not only able to detect the presence of a mutation but is also able to detect if this mutation is tumorous or healthy. This is indeed needed to improve personalized cancer treatment of a patient since a detection of a tumorous or healthy origin of a mutation will have different consequences on the therapeutic treatment of the patient. In addition, the method according to the present invention is able to work in an unsupervised manner, meaning that any mutation, even a previously unknown mutation, can be detected as tumorous or healthy. The method according to the present invention is also able to identify and detect a mutation from a liquid biopsy with a very high sensitivity, preventing the need to resort to deep sequencing, which reduces the cost of the method and allows a routine application of the method in clinical settings. Moreover, many known methods of DNA analysis of liquid biopsies rest on the use of unique molecular identifiers (UMIs) to improve the sensitivity of the methods. UMIs are short sequences added to DNA fragments during DNA library preparation of next generation sequencing protocols. These UMIs are specific tags to identify DNA fragments and are used to reduce errors introduced during PCR amplification before sequencing. Because the method according to the present invention analyzes the presence of DNA variant fragments in the first group and in the second group, it naturally compensates for the false positive due to clonal hematopoiesis. Different than in the state of the art, the present invention is based on the presence of a particular mutation in the two size groups of their DNA fragments, not mixing the different mutations in the two size groups. This was found to further increase the sensitivity of detecting the presence of a particular mutation and to be key for detecting, if the particular mutation is tumorous or healthy.

DNA fragments are fragments of DNA collected by the liquid biopsy. Preferably, a DNA fragment possesses a DNA sequence length comprises between 150 bp and 300 bp, but obviously the invention is not limited to these sizes and DNA sequence outside of this range can also be used for the present invention. The DNA fragments comprises DNA variant(s) fragments and/or DNA wild-type fragments. DNA wild-type fragments have a DNA sequence identical to the corresponding DNA sequence of a reference genome. Preferably, the DNA sequence of the reference genome is obtained by means of public database such as hg19 (for example: grch37) of NCBI. However, the reference genome can be also the genome originating from a healthy DNA sample of the patient. DNA variants fragments refer to or are all DNA fragments having at least one variation from the DNA sequence of the reference genome, i.e. comprise a mutation. The DNA variants fragments do not distinguish between different mutations and refer to all DNA fragments having any mutation. DNA variant fragments refer to or are all DNA fragments having one particular variation of the DNA (particular DNA variant), i.e. all DNA fragments having the same mutation. The term "mutation" refers to a particular variation in a DNA sequence of a DNA fragment as compared to the corresponding DNA sequence of a reference genome. Thus, the mutation refers to a certain variation of a certain portion of the DNA. The DNA variant fragments show all the same mutation in at least a portion of the DNA variant fragments. The DNA variant fragments can have different mutations in different portions of the DNA variant fragments. The same mutation can refer to the complete sequence of the DNA variant (fragment) (so that all DNA variant fragments would be realizations of the same DNA variant sequence) or to just a sub-portion of the DNA in which the DNA sequence has the same mutation (so that the DNA variant fragments are identical in said sub-portion of the DNA and could be different in other portions of the DNA). So, in the latter embodiment a DNA variant fragment having two mutations in two distinct sub-portions can be considered/identified for two distinct particular mutations. So, the combination of the DNA variant fragments of all mutations collected from the liquid biopsy form the DNA variants fragments. Preferably, the mutations belong to a group of variants comprising a single-nucleotide polymorphism, an indel, a deletion, a translocation, a copy number variation, or a combination thereof.

There is provided according to the present invention, a method to detect a mutation from a liquid biopsy of a patient, the method comprises the steps of:
- collecting DNA fragments from said liquid biopsy,
- preparing a DNA library of the DNA fragments from said liquid biopsy by preserving DNA fragments length,
- sequencing the DNA library and collecting sequencing data of the DNA fragments of the DNA library, wherein sequencing the DNA library is done by a targeted sequencing using a targeted panel, wherein the targeted panel either defines a plurality of mutations known to be tumorous in general or a plurality of mutations being identified from a sample of the patient taken prior to the liquid biopsy,
- based on the sequencing data, identifying DNA variant fragments of the DNA fragments having a same mutation,
- associating the identified DNA variant fragments to a first group, if the DNA variant fragments are shorter than a first intermediate length threshold, wherein the first intermediate length threshold is between 150 bp and 166 bp,
- associating the identified DNA variant fragments to a second group, if the DNA variant fragments are longer than a second intermediate length threshold, wherein the second intermediate length threshold is between 150 bp and 166 bp, wherein preferably the first intermediate length threshold and the second intermediate length threshold are equal,
- detecting, if the mutation is tumorous or healthy based on the presence of the DNA variant fragments in the first group and in the second group, wherein the mutation is detected as tumorous, if the DNA variant fragments are present more often in the first group than in the second group.

There is also provided according to the present invention, a method to identify, to follow and/or to adapt a treatment adapted to a personalized mutation profile of a tumor of a patient, said method comprising the steps of:
- collecting DNA from a tumorous sample of the patient,
- sequencing said DNA from the tumorous sample,
- identifying mutations from said sequencing,
- designing a panel of specific DNA probes targeting the identified mutations,
- performing the method according to anyone of the previous claims, wherein the DNA fragments of a liquid sample of the patient are collected with the designed panel,
- characterizing the personalized mutation profile of the tumor based on the detection of tumorous and/or healthy mutations.
The present invention also relates to a theranostic report of a tumor of a patient created based on the method to identify, to follow and/or to adapt a treatment adapted to a personalized mutation profile of a tumor of a patient according to the present invention.

According to the present invention, it is indeed possible to detect if a DNA variant from a liquid biopsy of a patient is tumorous or healthy. To be able to establish with confidence a personalized mutation profile of the tumor of the patient from a liquid biopsy, it is indeed not only needed to detect mutations with higher sensitivity, but it is also important to be able to detect if a mutation originates from a tumor cell or from a normal, healthy, cell of the patient. According to the present invention, it is also possible to create a theranostic report of the patient which incorporates the characteristics of the tumor of the patient. Such report is very useful, for example in a clinical setting for medical doctors to be able to take the best possible medical decisions according to the information available about the tumor of the patient.

Dependent claims refer to other advantageous embodiments.

Preferably, the detection whether a mutation is tumorous or healthy is based on the presence of the DNA variant fragments in the first group and in the second group and based on the mutation frequency of the mutation. It is indeed useful to cluster the mutation as healthy or tumorous based on the length of the DNA variant fragment having the mutation and based on the DNA mutation frequency.

Advantageously, a mutation is detected as tumorous, if the DNA variant fragments are present more often in the first group than in the second group and if the mutation has a mutation frequency below a frequency threshold, wherein the frequency threshold is between 2% and 50%.

Preferably, the DNA variant fragments are associated to the second group, if the DNA variant fragments are longer than the second intermediate length threshold and shorter than an upper length threshold. Indeed, such first and upper length thresholds allow a proper clustering of a first group and a second group, which is needed to discriminate between healthy and tumorous DNA variant.

Preferably, the targeted sequencing is a hybridization targeted sequencing with double-stranded DNA probes. Indeed, the inventors have found that a hybridization step with double-stranded DNA probes is more efficient to capture a DNA of interest for targeted sequencing, allowing for a uniform capture of the DNA of interest and thus for a uniform sequencing of the DNA of interest.

Advantageously, the DNA library is a single-stranded and a double-stranded DNA library. Indeed, the presence of both a single-stranded and a double-stranded DNA library permits an enrichment of the tumorous DNA fragments as compared to the normal, healthy, DNA fragments, which improves the sensitivity of the detection of tumorous DNA variants.

According to the present invention, the term tumorous DNA fragments refers to DNA fragments originating from a tumor cell and/or from a tumor of the patient.

Preferably, the targeted sequencing is preferably done with a personalized panel defining the mutations identified in the tumor of the patient. Preferably, the targeted sequencing is a targeted sequencing of a DNA sequence having a total length of at least 0,5 Mb, preferably at least 0,8 Mb, preferably at least 1 Mb of the DNA of the tumor of the patient, wherein the DNA sequence having said total length may be a series of continuous and/or discontinuous DNA sequences having a cumulative total length of at least 0,5 Mb, preferably at least 0,8 Mb, preferably at least 1 Mb of the DNA of the tumor of the patient. The total length of the DNA sequence sequenced refers to the cumulative number of base pairs read (accumulated also over different DNA "pieces" read). The invention is particularly advantageous with personalized panels, because first the mutations appearing in a tumor of a patient are identified and defined in the targeted/personalized panel. In addition, the method to detect a mutation from a liquid biopsy of a patient with a personalized panel, does not only detect the presence of the defined, personalized, mutations, but also allows to distinguish between the tumorous and healthy (or non-tumorous) mutations. The combination of a personalized panel with the inventive method to detect a mutation from a liquid biopsy of a patient is a very powerful tool for cancer surveillance. Alternatively, or in addition, the targeted sequencing can also be done with a targeted panel defining known DNA variants frequently associated to a tumor type of the patient and/or known DNA variants associated to a response or resistance to a treatment of said tumor type of the patient.

Advantageously, the method comprises the step of performing a sequencing of a tumorous sample of the patient to identify the mutations of the personalized or targeted panel. Preferably, an identification of a mutation from the first group and/or from the second group is performed by a comparison of the mutation in the first and second group where: (i) the mutation is detected as a healthy mutation if said mutation is present only in the second group, and/or (ii) the mutation is detected as a healthy mutation if a DNA mutation frequency of the DNA mutation is superior to the frequency threshold, and/or (iii) the mutation is detected as a healthy mutation if said mutation is present more often in the second group than in the first group. Indeed, besides detecting tumorous mutations, it is also advantageous to detect healthy mutation to better define the tumor of the patient.

Advantageously, the liquid biopsy is performed on a patient in at least one of the following stages: (i) a patient having a tumor before a treatment, (ii) a patient having a tumor during a treatment, (iii) a patient having a tumor after a treatment is finished, (iv) a patient in a tumor remission phase, (v) a patient in a tumor relapsed phase. Indeed, to better follow an evolution of the tumor of the patient and/or to better analyze the efficacy of a tumor treatment, it is useful to perform the methods according to the present invention on a DNA sample obtained from the liquid biopsy, wherein the liquid biopsy is performed at different stages of the tumor evolution of the patient and/or at different stages of treatment of the patient. Especially, in the tumor relapse phase, the method is particularly advantageous as the relapse of the tumor can be detected with a high sensitivity, but with a low false positive rate.
Preferably, for each respective mutation of the plurality of mutations defined in the targeted panel the following steps are performed:
- based on the sequencing data, identifying DNA variant fragments of the DNA fragments having the respective mutation,
- associating the identified DNA variant fragments of the respective mutation to a first group, if the DNA variant fragments of the respective mutation are shorter than a first intermediate length threshold, wherein the first intermediate length threshold is between 150 bp and 166 bp,
- associating the identified DNA variant fragments of the respective mutation to a second group, if the DNA variant fragments of the respective mutation are longer than a second intermediate length threshold, wherein the second intermediate length threshold is between 150 bp and 166 bp, wherein preferably the first intermediate length threshold and the second intermediate length threshold are equal,
- detecting, if the respective mutation is tumorous or healthy based on the presence of the DNA variant fragments of the respective mutation in the first group and in the second group, wherein the respective mutation is detected as tumorous, if the DNA variant fragments are present more often in the first group than in the second group.

Advantageously, the liquid sample originates from the patient in a tumor remission phase. Indeed, it is advantageous to perform the methods according to the present invention in a patient in a tumor remission phase to analyze, in a non-invasive manner, a potential re-emergence of the tumor of the patient.

Preferably, an identification, a follow-up and/or an adaptation of the treatment is adapted to the characterization of the personalized mutation profile of the tumor according to the present invention. Indeed, the identification, the follow-up and/or the adaptation of the treatment will benefit from the characterization of the personalized mutation profile of the tumor of the patient.

Advantageously, a treatment adapted to the characterization of the personalized mutation profile of the tumor of the patient is applied to said patient.

Other features, details and benefits of the invention will emerge from the description given below, non-limitingly and referring to the attached drawings.
Figure 1 is a flow chart explaining the steps to design a targeted panel of specific DNA probes.
Figure 2 shows the method to detect a mutation from a liquid biopsy of a patient according to the invention and the method to identify, to follow and/or to adapt to a treatment adapted to a personalized mutation profile of a tumor of a patient according to the invention.
Figure 3 illustrates how a DNA variant fragment fulfills the conditions of a first group or a second group.
Figure 4 shows a scheme illustrating the clustering of DNA variant fragments having a first mutation into a first group and a second group.
Figure 5 shows a scheme illustrating the clustering of DNA variant fragments having a second DNA mutation into a first group and a second group.
Figure 6 is a flow chart explaining the steps to identify a mutation as tumorous or healthy.
Figure 7 is a scheme showing that a liquid biopsy can be performed at different stages of tumor evolution and/or clinical treatment of the patient.

In the drawings, the same reference numbers have been allocated to the same or analogue element.

Other characteristics and advantages of the present invention will be derived from the non-limitative following description, and by making reference to the drawings and the examples

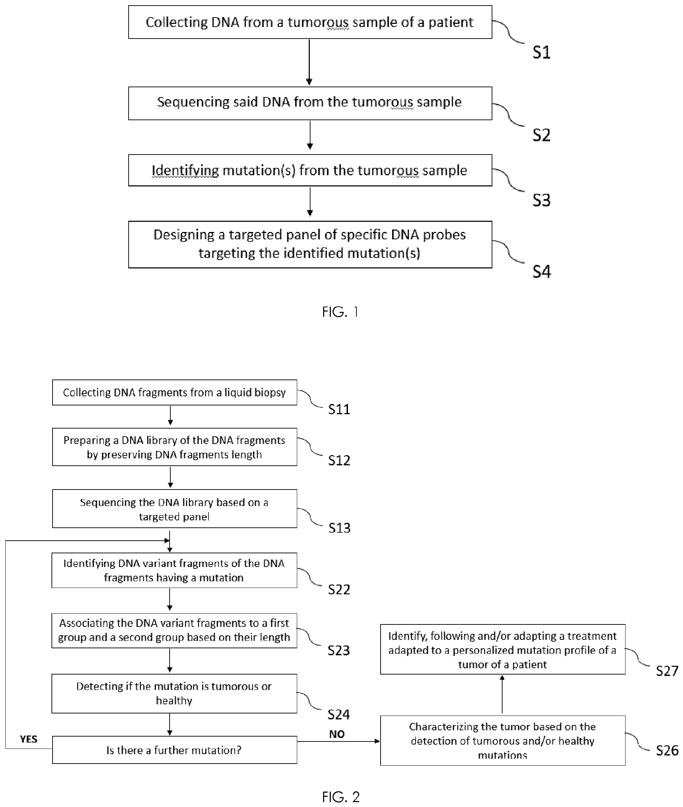

Figure 1 illustrates the different steps to design a targeted panel of specific DNA probes from a collection of DNA from a tumorous sample of a patient.

A first step S1 consists of collecting DNA from a tumorous sample of a patient. In some implementations, the tumorous sample of the patient originates from a solid biopsy. In some implementations, the tumorous sample of the patient originates from a liquid biopsy. The DNA from a tumorous sample of the patient can be DNA fragments from the tumor and/or genomic, chromosomic, DNA from the tumor.

In step S2, said DNA from the tumorous sample is sequenced. Preferably, sequencing said DNA is a targeted sequencing of at least 100.000 base pairs (bp) or 100 kilo bp (kb), preferably of at least 500 kb, preferably of at least 700 kb, preferably of at least 800 kb, preferably of at least 900 kb, preferably of at least 1.000 kb or 1 mega bases (Mb) of said DNA from the tumorous sample of the patient. Preferably, sequencing said DNA is a targeted sequencing of less than 10 Mb, preferably of less than 5 Mb, preferably of less than 3 Mb of said DNA from the tumorous sample of the patient. Advantageously, sequencing is performed by a NextSeq500/550 sequencer or any Illumina or MGI sequencer. Preferably, sequencing is a paired end sequencing 'paired end sequencing' but can also be a single end sequencing 'single end sequencing'.

Then, identifying mutations from the tumorous sample is performed in step S3. The identification of mutations is identified based on a comparison of the sequencing DNA from the tumorous sample with the corresponding DNA sequence of the reference genome.

A next step S4 consists of designing a targeted panel of specific DNA probes targeting the identified mutations.

Figure 2 discloses an embodiment of a method to detect a mutation from a liquid biopsy of a patient according to the invention and of a method to identify a treatment adapted to a mutational profile of a tumor of a patient. While steps S11 to S13 describes rather the laboratory steps of the method(s), steps 21 to 27 describe rather the in-silico steps. However, it is also possible that some steps of steps S11 to S13 can be performed in-silico and/or that one or more steps of steps 21 to 27 are performed not in-silico.

A step S11 consists of collecting DNA fragments from the liquid biopsy.

A next step S12 consists of preparing a DNA library of the DNA fragments by preserving DNA fragments length. Advantageously, the DNA library is prepared based on DNA fragments comprising single-stranded and/or double-stranded DNA fragments. Preferably, the DNA library is prepared based on DNA fragments comprising single-stranded and double-stranded DNA fragments.

Then, sequencing the DNA library based on a targeted panel is performed in S13. The targeted panel defines a plurality of mutations. The targeted panel allows to sequence the DNA fragments in the liquid biopsy or library having the defined mutations. Thus, the sequencing data resulting from sequencing the DNA library based on the targeted panel will read out or sequence all DNA fragments having the mutations defined in the targeted panel (while the DNA fragments not showing the defined mutations are not read out or sequenced). The targeted panel can also define the wild-type DNA so that the targeted panel will also sequence the DNA fragments without mutations. The resulting sequencing data contain thus the DNA sequence of the DNA fragments of the liquid sample having the defined mutations (and maybe belonging to the wild-type DNA). Preferably, the targeted panel is a panel targeted to the cancer of the patient, i.e. a patient specific panel which is designed based on a sample of the patient. Such a patient specific targeted panel can be made as described in Figure 1. Advantageously, the targeted sequencing is performed to enrich DNA having (i) known mutations frequently associated to a tumor type of the patient, and/or (ii) known mutations associated to response or resistance to a treatment of said tumor type of the patient, and/or (iii) personalized mutations identified in the tumor of the patient. However, the targeted panel can also be non-patient specific. The target panel can for example include a plurality of mutations known to cause a certain cancer type, i.e. a cancer specific panel. The certain cancer type can be for example a lung cancer, a breast cancer, skin cancer, etc. The targeted sequencing is a hybridization targeted sequencing.

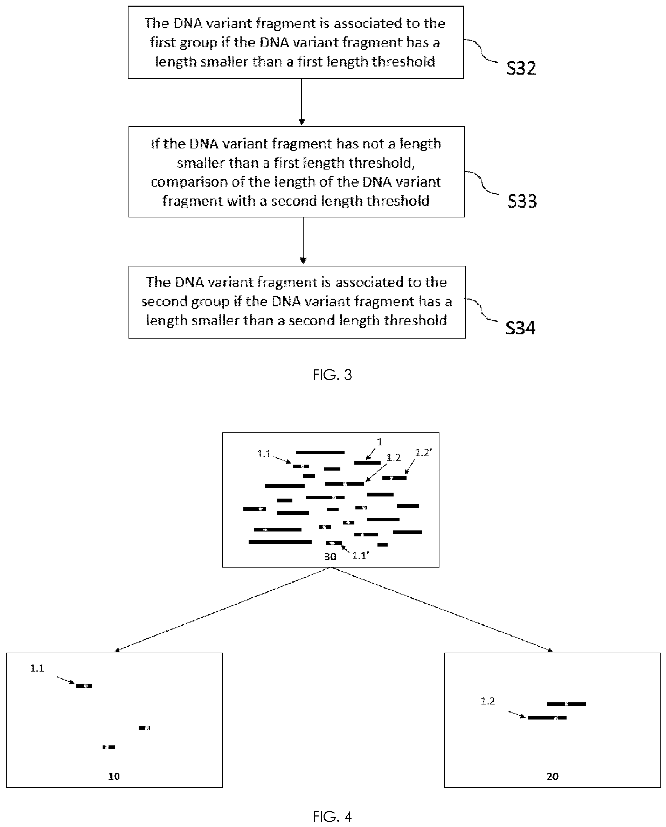

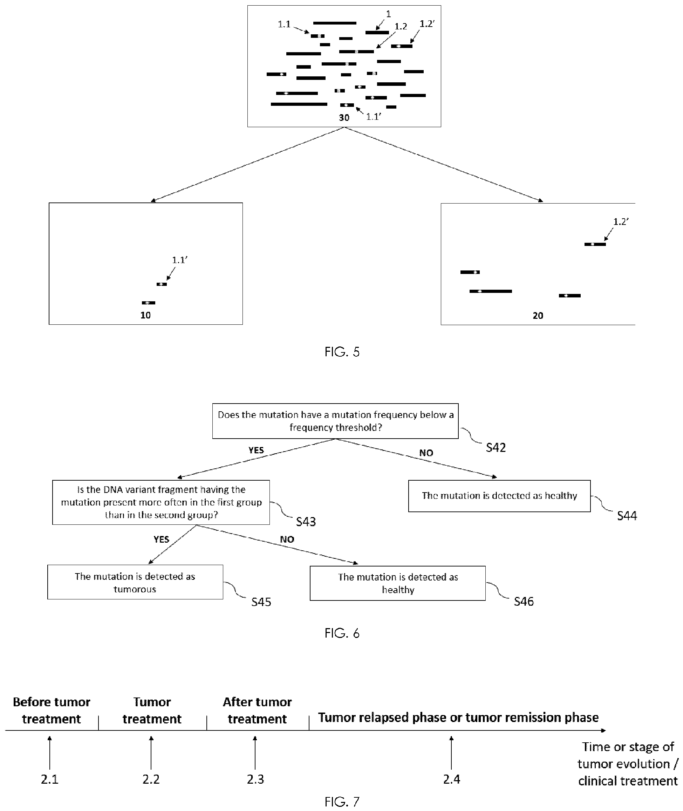

The sequencing step provides (digital) DNA sequencing data (short sequencing data). Preferably, the sequencing data provide, for each (sequenced) DNA fragment, the sequence information of this DNA fragment. This sequence information comprises preferably the nucleotide sequence of the (sequenced) DNA fragment. Fig. 4 and 5 shows sequencing data 30 with the sequenced DNA fragments. For simplicity of the illustration, only wild-type DNA fragments 1, first DNA variant fragments 1.1, 1.2 having a first certain mutation (shown as a grey rectangle) and second DNA variant fragments 1.1', 1.2' having a second certain mutation (shown as a white circle). The example shall not limit the invention, and the sequencing data can comprise the DNA variants fragments of more than two different mutations.

In step S22, DNA variant fragments having a certain mutation are identified in the DNA fragments of the DNA sequencing data. That means that in step S22, the DNA variant fragments (having the certain mutation) are selected among all DNA fragments in the DNA sequencing data. This selection is preferably performed in-silico, i.e. is performed based on the digital sequencing data. Thus, the DNA variant fragments mentioned in the subsequent steps S23 and S24 refer thus always to the DNA variant fragments of the same certain mutation. In the example of Fig. 4, the DNA fragments of the first DNA variant fragment 1.1, 1.2 are identified/selected. In the example of Fig. 5, the DNA fragments of the second DNA variant fragment 1.1', 1.2' are identified/selected. Step S22 results thus in a set of DNA variant fragments, i.e. in a set of DNA fragments belonging to the same certain mutation.

In step S23, the identified DNA variant fragments are associated with a first group and with a second group based on the length of the DNA variant fragments. The DNA variant fragments of the first group are shorter than those of the second group, at least the majority of the DNA variant fragments of the first group are shorter than those of the second group. Preferably, all DNA variant fragments of the first group are shorter than all DNA variant fragments of the second group. DNA variant fragments of the first/second group shall mean DNA variant fragments associated with the first/second group. Step S23 results thus in a first set/group 10 of shorter DNA variant fragments and in a second set/group 20 of longer DNA variant fragments. The first and second group 10, 20 comprise only the DNA fragments of the certain mutation, i.e. of only one (and/or of the same) mutation. Preferably, the DNA variant fragments 1.1 being shorter than a first intermediate length threshold are associated to the first group 10, and the DNA variant fragments 1.2 being longer than a second intermediate length threshold are associated to the second group 20. Most preferably, the first intermediate length threshold and the second intermediate length threshold are the same, i.e. a (common) intermediate length threshold as will be shown in the example in Fig. 3. It is however also possible that the first intermediate length threshold and the second intermediate length threshold are different. In this case, the first intermediate length threshold and the second intermediate length threshold distinguish preferably by less than 5 base pairs (bp), preferably by less than 3 bp. The intermediate length threshold, the first intermediate length threshold and/or the second intermediate length threshold is/are preferably smaller than 167 bp, preferably smaller than 166 bp, preferably smaller than 165 bp, preferably smaller than 164 bp, preferably smaller than 163 bp, preferably smaller than 162 bp, preferably smaller than 161 bp and/or is preferably larger than 150 bp, preferably larger than 155 bp, preferably larger than 157 bp, preferably larger than 158 bp, preferably larger than 159 bp. In a preferred embodiment, the intermediate length threshold is 160 bp long. There can be additional criteria to associate DNA fragments to the first group 10 or to the second group 20. Preferably, the DNA variant fragments 1.2 being longer than the second intermediate length threshold (or the intermediate length threshold) and being shorter than an upper length threshold are associated to the second group 20. The upper length threshold is larger than the (second) intermediate length threshold. Preferably the upper length threshold is smaller than 250 bp, preferably than 200 bp, preferably than 190 bp, preferably than 185 bp. In a preferred embodiment, the upper length threshold is 180 bp.

Figure 3 illustrates an example how the (identified) DNA variant fragments are associated with the first and second group based on their length. This is realized by performing for each identified DNA variant fragment the method of Fig. 3. Thus, step S23 of Fig. 2 can be realized by performing for each (identified) DNA variant fragment the method shown in Fig. 3. If the DNA variant fragment is smaller than the intermediate length threshold, the DNA variant fragment fulfills the conditions of the first group 10 and/or is associated in step S32 to the first group 10. If the DNA variant fragment is not smaller or is larger than the intermediate length threshold, the process continues to step S33 in which it is further checked, if the DNA variant fragment is smaller than the upper length threshold. If this is the case (i.e. the DNA variant fragment has a length between the intermediate length threshold and the upper length threshold), the DNA variant fragment is associated in step S34 to the second group 20. The order of the steps and checks of steps S32 to S34 can be rearranged arbitrarily provided that the same DNA variant fragments are associated to the first and second group 10, 20 like in this procedure. So, as illustrated in Fig. 4, the shorter DNA variant fragments 1.1 are associated to the first group 10, while the longer DNA variant fragments 1.2 are associated to the second group 20.

The order of steps S22 and S23 is not important for the invention. Either the DNA variant fragments belonging to one mutation are first identified and then associated based on their length to the two groups 10, 20 or all DNA (variants) fragments are first associated based on their length to the two groups 10, 20 and then the DNA variant fragments belonging to the one mutation are identified in the first and second group 10, 20. When stating that the identified DNA variant fragments are associated to the first group 10 and/or to the second group 20, it shall not imply that the identification step S22 is performed necessarily before the association step S23.

In step S24, it is detected, if the certain mutation (of the DNA variant fragments) is tumorous or healthy. This detection is preferably based on the presence of the DNA variant fragments of the certain mutation in the first and second group 10, 20. Preferably, the certain mutation is preferably detected as tumorous, if more DNA variant fragments (of the certain mutation) are present in the first group 10 than in the second group 20, preferably statistically significant more than in the second group 20. This detection is preferably based on mutation frequency. This detection is preferably based on the presence of the DNA variant fragments of the certain mutation in the first and second group 10, 20 and based on the mutation frequency. Preferably, the certain mutation is detected as tumorous, if the mutation fulfills cumulatively the two conditions that the mutation frequency of the mutation is below a frequency threshold and that more DNA variant fragments (of the certain mutation) are present in the first group 10 than in the second group 20, preferably statistically significant more than in the second group 20. The statistical significance can for example be tested with a chi square test.

The mutation frequency is often also called the allele frequency or the gene frequency. The mutation frequency is determined based on the frequency of the certain mutation in the liquid biopsy and/or in the sequencing data retrieved from the liquid biopsy. The mutation frequency of the mutation can be determined based on a ratio of the presence of the mutation and of the presence of the wild-type DNA. Preferably, this is determined by ratio #V/#R of the number #V of DNA variant fragments in the sequencing data and of the number #R of DNA fragments in the sequencing data. The number of DNA fragments in the sequencing data #R corresponds to the number of reads, i.e. the number of all DNA fragments read with the sequencing process. However, other definitions are also possible. For example, the mutation frequency could be also defined by the corresponding ratio defined before within a certain size range of the DNA fragments or other definitions representing the mutation frequency. The frequency threshold is preferably larger than 5 percent (%), preferably larger than 10 %, preferably larger than 15 %, preferably larger than 18%. The frequency threshold is preferably smaller than 40 %, preferably smaller than 30%, preferably smaller than 25%, preferably smaller than 22%. In a preferred embodiment, the frequency threshold is 20%.

Preferably, the mutation is detected as healthy or non-tumorous, if the above defined condition(s) for a tumorous mutation is not fulfilled and/or if the mutation frequency of the mutation is larger than the frequency threshold and/or if there are more DNA variant fragments in the second group 20 than in the first group 10. However, it is also possible that the method determines just, if the mutation is tumorous or not. In this case, the detection that the mutation is not detected as tumorous is for the patent considered as the mutation is detected as healthy.

Figure 6 is an exemplary flow chart explaining how a mutation is detected as tumorous or healthy. Fig. 6 is thus an example method for realizing step S24 in Fig. 2. In step S42, a check is performed to see, if the mutation has a mutation frequency below the frequency threshold. In S44, the mutation is detected as healthy, if the mutation does not have a mutation frequency below the frequency threshold. If the mutation has a mutation frequency below the frequency threshold, a check is performed in S43 to see if the DNA variant fragments are present more often in the first group 10 than in the second group 20. If the DNA variant fragments having the mutation are more often present in the first group 10 than in the second group 20, the mutation is detected as tumorous in step S45, while if the DNA variant fragments having the mutation are not more often present in the first group 10 than in the second group 20 and/or are more often present in the second group 20 than in the first group 10, the mutation is detected as healthy in step S46.

The method according to the invention can be applied for just one mutation. In this case, the method ends after step S24 or continues with step S27. Preferably, the method according to the invention is applied for a plurality of mutations so that for each of the plurality of mutations, it is detected, if the DNA variant is healthy or tumorous. Each of the plurality of mutations are defined in the targeted panel. In this case, the steps S22 to S24 are repeated for each mutation of the plurality of mutations so that for each of the plurality of mutations it can be determined in S24, if the mutation is tumorous or healthy. When all of the mutations of the plurality of mutations were processed, the method continues with step S26. While Fig. 4 shows the steps of identifying first DNA variant fragments S22 of a first mutation among the DNA fragments 30 and associating the first DNA variant fragments to the first group 10 and the second group S23, Fig. 5 shows the steps of identifying second DNA variant fragments S22 of a second mutation among the DNA fragments 30 and associating the second DNA variant fragments to the first group 10 and the second group S23. The first mutation is different from the second mutation.

Step S26 is optional and consists in characterizing the tumor based on the detection result of step S24 of the mutation or the mutations of the plurality of mutations. Characterizing the tumor based on the detection of the mutations that are tumorous and/or healthy means, for example, to identify a tumor sub-type that is particularly known to be sensitive and/or resistant to a particular tumor treatment. As a consequence, characterizing the tumor permits the identification of a particular tumor treatment that is well adapted to the tumor of the patient.

Also step S27 is optional and consists of identifying, following and/or adapting a treatment adapted to a personalized mutation profile of the tumor of the patient based on the characterization of the tumor of step S26 or based on the detection result(s) of step S24. Preferably, the theranostic report of the tumor of the patient may comprise medical information and/or sequencing data of the tumor with a list of mutations identified that are tumorous and/or healthy. The theranostic report of the tumor may also comprise (i) data for a potential of response to a therapy based on the characterization of the tumor, (ii) a list of treatments with their properties that may be associated with a clinical benefit and/or those that are not associated with a clinical benefit, (iii) a list of clinical assays associated with the characterization of the tumor and/or a list of scientific publications linked to the characterization of the tumor.

Figure 7 shows different time points at which the liquid biopsy can be performed on the patient in order to monitor tumor evolution. Preferentially, the liquid biopsy is performed on a patient in at least one of the following stages: (i) a patient having a tumor before a treatment 2.1, (ii) a patient having a tumor during a treatment 2.2, (iii) a patient having a tumor after a treatment is finished 2.3, (iv) a patient in a tumor remission phase 2.4, (v) a patient in a tumor relapsed phase 2.4. Preferably, the liquid biopsy is taken from the patient in a tumor remission phase.
It should be understood that the present invention is not limited to the described embodiments and that variations can be applied without going outside of the scope of the claims.

## Claims

1. A method to detect a mutation from a liquid biopsy of a patient, the method comprises the steps of:
- collecting DNA fragments from said liquid biopsy,
- preparing a DNA library of the DNA fragments from said liquid biopsy by preserving DNA fragments length,
- sequencing the DNA library and collecting sequencing data of the DNA fragments of the DNA library, wherein sequencing the DNA library is done by a targeted sequencing using a targeted panel, wherein the targeted panel either defines a plurality of mutations known to be tumorous in general or a plurality of mutations being identified from a sample of the patient taken prior to the liquid biopsy,
- based on the sequencing data, identifying DNA variant fragments of the DNA fragments having a same mutation,
- associating the identified DNA variant fragments to a first group, if the DNA variant fragments are shorter than a first intermediate length threshold, wherein the first intermediate length threshold is between 150 bp and 166 bp,
- associating the identified DNA variant fragments to a second group, if the DNA variant fragments are longer than a second intermediate length threshold, wherein the second intermediate length threshold is between 150 bp and 166 bp, wherein preferably the first intermediate length threshold and the second intermediate length threshold are equal,
- detecting, if the mutation is tumorous or healthy based on the presence of the DNA variant fragments in the first group and in the second group, wherein the mutation is detected as tumorous, if the DNA variant fragments are present more often in the first group than in the second group.

2. The method according to claim 1, wherein it is detected, if the mutation is tumorous or healthy, based on the presence of the DNA variant fragments in the first group and in the second group and based on the mutation frequency of the mutation.

3. The method according to anyone of the previous claims, wherein the mutation is detected as tumorous, if the DNA variant fragments are present more often in the first group than in the second group and if the mutation has a mutation frequency below a frequency threshold, wherein the frequency threshold is between 2% and 50%.

4. The method according to anyone of the previous claims, wherein the DNA variant fragments are associated to the second group, if the DNA variant fragments are longer than the second intermediate length threshold and shorter than an upper length threshold.

5. The method according to anyone of the previous claims, wherein the DNA library is a single-stranded and a double-stranded DNA library.

6. The method according to anyone of the preceding claims, wherein an identification of a mutation from the first group and/or from the second group is performed by a comparison of the mutation in the first and second group where:
(i) the mutation is detected as a healthy mutation if said mutation is present only in the second group, and/or
(ii) the mutation is detected as a healthy mutation if a DNA mutation frequency of the DNA mutation is superior to the frequency threshold, and/or
(iii) the mutation is detected as a healthy mutation if said mutation is present more often in the second group than in the first group.

7. The method according to anyone of the preceding claims, wherein said liquid biopsy is performed on a patient in at least one of the following stages: (i) a patient having a tumor before a treatment, (ii) a patient having a tumor during a treatment, (iii) a patient having a tumor after a treatment is finished, (iv) a patient in a tumor remission phase, (v) a patient in a tumor relapsed phase.

8. The method according to anyone of the previous claims, wherein for each respective mutation of the plurality of mutations defined in the targeted panel the following steps are performed:
- based on the sequencing data, identifying DNA variant fragments of the DNA fragments having the respective mutation,
- associating the identified DNA variant fragments of the respective mutation to a first group, if the DNA variant fragments of the respective mutation are shorter than a first intermediate length threshold, wherein the first intermediate length threshold is between 150 bp and 166 bp,
- associating the identified DNA variant fragments of the respective mutation to a second group, if the DNA variant fragments of the respective mutation are longer than a second intermediate length threshold, wherein the second intermediate length threshold is between 150 bp and 166 bp, wherein preferably the first intermediate length threshold and the second intermediate length threshold are equal,
- detecting, if the respective mutation is tumorous or healthy based on the presence of the DNA variant fragments of the respective mutation in the first group and in the second group, wherein the respective mutation is detected as tumorous, if the DNA variant fragments are present more often in the first group than in the second group.

9. A method to identify, to follow and/or to adapt a treatment adapted to a personalized mutation profile of a tumor of a patient, said method comprising the steps of:
- collecting DNA from a tumorous sample of the patient,
- sequencing said DNA from the tumorous sample,
- identifying mutations from said sequencing,
- designing a panel of specific DNA probes targeting the identified mutations,
- performing the method according to anyone of the previous claims, wherein the DNA fragments of a liquid sample of the patient are collected with the designed panel,
- characterizing the personalized mutation profile of the tumor based on the detection of tumorous and/or healthy mutations.

10. The method according to the previous claim, wherein the liquid sample results from the patient in a tumor remission phase.

11. The method according to claim 9 or claim 10, wherein an identification, a follow-up and/or an adaptation of the treatment is adapted to the characterization of the personalized mutation profile of the tumor.

12. The method according to anyone of the claims 9 to 11, wherein a treatment adapted to the characterization of the personalized mutation profile of the tumor of the patient is applied to said patient.

13. The method according to anyone of the claims 9 to 12, wherein the liquid sample results from the patient in a tumor remission phase.

14. A theranostic report created based on the method according to anyone of the claims 9 to 13.
